# EUROPEAN PATENT SPECIFICATION

(11) **EP 3 126 382 B1**
(45) Date of publication and mention of the grant of the patent: **12.06.2019**
(21) Application number: 15722255.5
(22) Date of filing: 31.03.2015
(51) Int. Cl.: C07K 14/435, C07K 14/78, A23J 1/04

(54) **METHOD TO OBTAIN COLLAGEN/GELATIN FROM MARINE SPONGES**
VERFAHREN ZUR GEWINNUNG VON KOLLAGEN/GELATINE AUS MEERESSCHWÄMMEN
PROCÉDÉ D'OBTENTION DE COLLAGÈNE/GÉLATINE À PARTIR D'ÉPONGES MARINES

(30) Priority: 31.03.2014 PT 14107551
(43) Date of publication of application: 08.02.2017
(73) Proprietor: Association for the Advancement of Tissue Engineering and Cell Based Technologies & Therapies (A4TEC) - Associação, 4710-057 Braga (PT)
(72) Inventor: GONÇALVES DOS REIS, Rui Luis, P-4710-057 Braga (PT); CRUZ DUARTE, Ana Rita, P-4715-319 Braga (PT); COLARDELLE DA LUZ MANO, João Filipe, P-4715-050 Braga (PT); QUINTEIROS LOPES HENRIQUES DA SILVA, Tiago José, P-4990-240 Ponte de Lima (PT); DE ASCENSÃO AROSO, Ivo Manuel, P-4455-435 Perafita (PT); ANTUNES BARROS, Alexandre António, P-4700-737 Palmeira Braga (PT); FERNANDES DA SILVA, João Carlos, P-1900-097 Lisboa (PT)
(74) Representative: Patentree
(86) International application number: PCT/IB2015/052370
(87) International publication number: WO 2015/151030

(56) References cited:
- WO-A2-2006/089660
- SWATSCHEK D ET AL: "Marine sponge collagen: isolation, characterization and effects on the skin parameters surface-pH, moisture and sebum", EUROPEAN JOURNAL OF PHARMACEUTICS AND BIOPHARMACEUTICS, ELSEVIER SCIENCE PUBLISHERS B.V., AMSTERDAM, NL, vol. 53, no. 1, 1 January 2002 (2002-01-01), pages 107-113, XP004331337, ISSN: 0939-6411, DOI: 10.1016/S0939-6411(01)00192-8
- GOMEZ-GUILLEN M C ET AL: "Extraction of gelatin from fish skins by high pressure treatment", FOOD HYDROCOLLOIDS, ELSEVIER BV, NL, vol. 19, no. 5, 1 September 2005 (2005-09-01), pages 923-928, XP027795244, ISSN: 0268-005X [retrieved on 2005-09-01]
- ANA RITA ET AL: "Green solvents extraction of sponge-origin collagen/gelatin for biomedical applications", 2014 ANNUAL MEETING & EXPOSITION, SOCIETY FOR BIOMATERIALS, 16 April 2014 (2014-04-16), XP055199286,
- J C Silva ET AL: "Collagen/gelatin extraction from marine sponges --- a green technology approach", Term Stem 2014, 23 October 2014 (2014-10-23), XP55197846, Retrieved from the Internet: URL:http://www.researchgate.net/profile/Jo ao_Silva61/publication/275893798_Collageng elatin_extraction_from_marine_sponges_-_a_ green_technology_approach/links/5549803f0c f2ebfd8e3b0785.pdf?disableCoverPage=true [retrieved on 2015-06-23]
- J C Silva ET AL: "MARINE SPONGES - AN ALTERNATIVE COLLAGEN/GELATIN SOURCE FOR BIOMEDICAL APPLICATIONS", Biomacromolecules European Journal of Pharmaceutics and Biopharmaceutics Tissue Engineering Part C Methods, 1 November 2014 (2014-11-01), pages 3452-3457, XP55197843, Retrieved from the Internet: URL:http://www.3bs.uminho.pt/system/files/ biblio/18294-Abstract_ICVS_3BsMeeting_Joao Silva.pdf [retrieved on 2015-06-23]
- ALEXANDRE A. BARROS ET AL: "Water and Carbon Dioxide: Green Solvents for the Extraction of Collagen/Gelatin from Marine Sponges", ACS SUSTAINABLE CHEMISTRY & ENGINEERING, vol. 3, no. 2, 2 February 2015 (2015-02-02), pages 254-260, XP055197933, US ISSN: 2168-0485, DOI: 10.1021/sc500621z
- SOUROUR ADDAD ET AL: "Isolation, Characterization and Biological Evaluation of Jellyfish Collagen for Use in Biomedical Applications", MARINE DRUGS, vol. 9, no. 12, 7 December 2011 (2011-12-07), pages 967-983, XP55197859, ISSN: 1660-3397, DOI: 10.3390/md9060967
- Z. ZHANG ET AL: "PHYSICOCHEMICAL PROPERTIES OF COLLAGEN, GELATIN AND COLLAGEN HYDROLYSATE DERIVED FROM BOVINE LIMED SPLIT WASTES", JOURNAL OF THE SOCIETY OF LEATHER TECHNOLOGISTS AND CHEMISTS, vol. 90, 1 January 2005 (2005-01-01), pages 23-28, XP055197642,

## Description

### Technical field

The present solution relates to a process to obtain collagen/gelatin based on water acidified with carbon dioxide for the isolation and/or purification of soluble compounds.

### Background

Traditionally, marine collagen/gelatin can be isolated from marine resources after acid, base, or enzymatic treatments. For sponges, a treatment with a complex Tris-HCl buffer solution (pH 9.5, 10 mM EDTA, 8 M urea, and 100 mM 2-mercaptoethanol) is commonly proposed (S watschek, D.; Schatton, W.; Kellermann, J.; Kreuter, J. R. Eur. J.Pharm. Biopharm. 2002, 53, 107-113), but such methods are generally time consuming as they involve several operating steps and have low selectivity and low extraction yields. Furthermore, they require the use of large amounts of solvents. Environmental concerns and strict legislation on the use of volatile organic solvents are forcing chemical industries to move toward the application of alternative processing methodologies, which comply with the green chemistry philosophy. Chemical industries have been moving toward the development of innovative processes as the awareness that sustainable development is becoming mandatory and essential for their competitiveness.

Different papers in the literature report the possibility to solubilize a fraction of collagen from different sources in acidic solutions, most of which are dilute acetic acid solutions (Zhang, Z. K.; Li, G. Y.; Shi, B. J. Soc. Leather Technol. Chem. 2006, 90, 23-28). The reported procedures, however, do not lead to high yields of extraction (between 0.4 and 2%), and the process requires several post operation steps for the purification of the products.

In the past decade, comprehensive manuscripts (Highberger, J. H. J. Am. Chem. Soc. 1939, 61, 2302-2303) indicate that sponges (*Porifera*) are the most promising avenue for blue biotechnology, and their leading role within marine biotechnology stems from their long evolutionary history. Some of these sponges are particularly rich in collagen, which is one of the most important and abundant proteins in the human body, with more than 20 genetically distinct forms known today.

The current industrial demand for collagen is up to 326.000 tons per year for different fields of application, including alimentary, cosmetics, pharmaceutical, and biomedical.

The advantages of the use of collagen include the fact that it is highly abundant in nature, nonantigenic, biodegradable, nontoxic, and biocompatible. However, there are some constrains hindering the development of new products. Nowadays, 98% of the collagen is of mammalian origin, for instance, from calf skin and bone. These carry a high risk of disease transmission such as bovine spongiform encephalopathy, as well as social and/or religious constraints. Both Judaism and Islam forbid the consumption of porcine-derived products, while Hindus do not consume bovine-related products.

These facts are disclosed in order to illustrate the technical problem addressed by the present disclosure.

### General description

Marine origin collagen/gelatin has been reported to be a great promise, but so far, there has been a gap between the scientific interest and the wide industrial application of this source of collagen/gelatin, because the collagen extraction yields are very low.

Sustainable development relies not only on the development of novel processes but also on the use of alternative sources of raw materials, which decrease dependence from fossil fuel resources. In this sense, the sea provides a plentiful resource of potential new products for society including biomaterials.

Aiming to overcome these drawbacks, alternative sources have been suggested, such as collagen from marine sources, including jellyfish, fish scales (S watschek, D.; Schatton, W.; Kellermann, J.; Kreuter, J. R. Eur. J.Pharm. Biopharm. 2002, 53, 107-113) and skin, cuttlefish skin, and starfish. Particularly, sponge collagen has unique physicochemical properties (Pozzolini, M.; Bruzzone, F.; Berilli, V.; Mussino, F.; Cerrano, C.;Benatti, U.; Giovine, M. Mar. Biotechnol. 2012, 14, 281-293) and is a promising resource, but sponge collagen is not available in large quantities because of the lack of efficient extraction methodologies.

One of the objectives of the disclosure subject matter is a new extraction process, using water and carbon dioxide as solvents with a natural origin raw material to obtain sponge origin collagen/gelatin. The disclosure subject matter relates to a new extraction methodology for the extraction of acid soluble collagen/gelatin, which normally denatures at high temperatures (human collagens) under mild operating conditions, in which water is acidified with carbon dioxide to promote the extraction of collagen/gelatin from different marine sponge species. This new process has surprisingly a higher collagen extraction yield.

Marine sponges are extremely rich in natural products and are considered a promising biological resource. The disclosure subject matter is related to a green extraction process with a natural origin raw material to obtain sponge origin collagen/gelatin for biomedical applications. Marine sponge collagen has unique physicochemical properties, but its application is hindered by the lack of availability due to inefficient extraction methodologies. Traditional extraction methods are time consuming as they involve several operating steps and large amounts of solvents. The present subject matter discloses a new extraction methodology under mild operating conditions in which water is acidified with carbon dioxide (CO₂) to promote the extraction of collagen/gelatin from different marine sponge species. Surprisingly, an extraction yield of approximately 50% of collagen/gelatin present in the raw material was achieved. The results of Fourier transformed infrared spectroscopy (FTIR), circular dichroism (CD), and differential scanning calorimetry (DSC) spectra suggest a mixture of collagen/gelatin with high purity, and the analysis of the amino acid composition has shown similarities with collagen from other marine sources. Additionally, in vitro cytotoxicity studies did not demonstrate any toxicity effects for three of the extracts.

In an embodiment, the materials used were sponge samples of the species *Thymosea* sp., *Chondrilla nuculla* and *Chondrosia reniformis.* The sponge samples of the species *Thymosea* sp. were collected in the Atlantic Ocean in the Azores. Samples of *Chondrilla nuculla* were collected in the Mediterranean Sea. *Chondrilla nuculla* from Alassio was grown in aquaculture, while *Chondrilla nuculla* from Portofino is the wild type. Samples of *Chondrosia reniformis* were collected on the Israeli coast.

In an embodiment, the collagen/gelatin extraction of the marine sponges *(Thymosea* sp. (TIM), *Chondrosia reniformis* (CR), *Chondrilla nuculla*, Alassio (ConAL), and *Chondrilla nuculla*, Portofino (ConPF)) were ground in small pieces, in particular five grams of sponge material was weighed and washed with distilled water overnight to remove salt and any contaminants present and were then lyophilized. Afterward, the samples were placed in a high pressure vessel (30 cm³), and distilled water was added, in particular 10 mL. The vessel was heated, in particular, to 37 °C. The system was pressurized with carbon dioxide, in particular, to 50 bar. The extraction was performed in particular, in batch mode for 16 h. After this time, the high pressure vessel was rapidly depressurized. The extract obtained was filtered with a 0.45 µm filter and frozen. Collagen/gelatin powder was obtained after freeze-drying of the extracts.

A high pressure device is a device capable of applying pressures greater than 100 bar. The pressure is applied uniformly to the sample by injection of carbon dioxide.

In an embodiment, the yield of extraction of the marine sponge collagen/gelatin was quantified as the ratio of dry extract obtained per weight of initial dry sample. The quantification of collagen/gelatin on the different extracts was performed using, in particular, the Sircol assay kit. The Sircol assay is a dye-binding method specific for the analysis of collagen/gelatin. The determination of collagen/gelatin was carried out according to the protocol described by the manufacturer (Sircol, Soluble Collagen Asay, Biocolor, Life Science Assays, U.K.).

In an embodiment, the powder obtained from the different extractions was analysed by a Nova NanoSEM 200 scanning electron microscope (SEM). The samples were fixed by mutual conductive adhesive tape on aluminium stubs and covered with gold using a sputter coater prior to microscopic analysis.

In an embodiment, the Fourier transform infrared spectroscopy (FTIR) spectra of the extracts were obtained with a Shimadzu-IR Prestige 21 spectrometer in the spectral region of 4000-800 cm⁻¹ with resolution of 2 cm⁻¹ as the average of 32 individual scans. The samples were analyzed in KBr pellets.

In an embodiment, the circular dichroism (CD) measurements of the extracted material were performed using, in particular, a Jasco Model J-865 spectropolarimeter (Jasco, U.K.) using a quartz cylindrical cuvette (Hellma, Germany) with a path length of 0.1 mm. The cuvette was filled with 1.50 mL of sample (0.01 g/mL) for each measurement. CD spectra were obtained by continuous wavelength scans (average of three scans) from 180 to 260 nm at a scan-rate of 50 nm/min. The samples were equilibrated for 1 h at room temperature before the CD spectra were acquired.

In an embodiment, the differential scanning calorimetry experiments were carried out using a DSC Q100 equipment (TA Instruments, U.S.A.). The experiments were conducted under a nitrogen atmosphere on samples (5-10 mg) packed in aluminium pans. The samples were heated at a constant heating rate of 5 °C/min from 0 to 90 °C, followed by an isothermal period at 90 °C. The samples were then cooled at the same rate to the initial temperature.

In an embodiment, the amino acid content was determined, in particular, by quantitative amino acid analysis using a Biochrom 30 (Biochrom Ltd., Cambridge, U.K.). Briefly, the samples were hydrolyzed and separated by an ion exchange column. After postcolumn derivatization by ninhydrin, the samples were analyzed at two wavelengths: 440 and 570 nm. An internal standard of norleucine was used to determine the concentrations of amino acids in the sample.

In an embodiment, the isoelectric point of the samples was determined following a titration protocol described in the literature (Etherington, D. J. Ann. Rheum. Dis. 1977, 36, 14-17), in particular for each extract, 10 mg of collagen/gelatin was dissolved in 6 mL of Milli-Q water. Each sample was titrated with a solution of 0.02N NaOH and with a solution of 0.02 N HCl. The pH was registered (pH meter, 3510 Jenway), and the resulting pH values were plotted versus the amount of NaOH and HCl. The titration range was from pH 2 to 12. All experiments were performed at room temperature.

In an embodiment, the determination of the molecular weight of the samples was carried out by size exclusion chromatography (GPC-SEC), in particular 1 mg of extract was dissolved in 1 mL of an aqueous solution of sodium nitrate 0.2 M containing 0.02% of sodium azide. The solutions were filtered through a 0.22 µm membrane and analysed on a size exclusion chromatograph (Viscotek TDA 305) equipped with three detectors: light scattering, refractive index, and viscometer. Elution was performed at 30 °C using a flow rate of 1 mL/min of the following eluent: aqueous solution of sodium nitrate 0.2 M containing 0.02% sodium azide. The column set was composed by a guard precolumn Aq. Guard col 50 mm × 6.0 mm (Viscotek) and a PLaquagel-OH mixed 8 µm (300 mm × 7.5 mm, Polymer Laboratories).

In an embodiment, the cytotoxicity of the extracts was assessed on bioreduction of a novel tetrazolium compound, 3-(4,5-dimethylthiazol-2-yl)-5-(3-carboxymethoxyphenyl)-2-(4-sulfofenyl)-2H-tetrazolium (MTS) (cell titer 96 aqueous solution cell proliferation assay, Promega, U.S.A.). MTS assay was measured in accordance with ISO/EN 10993 Part 5 guidelines27 using an immortalized mouse lung fibroblasts cell line (L929 cell line) purchased from the European Collection of Cell Cultures. In particular 1.5 × 10⁴ cells/mL were cultured in a 48 well plate in Dulbecco's modified Eagle's medium, supplemented with 10% fetal bovine serum (Alfagene, U.S.A.) and 1% antibiotic/antimycotic solution (Gibco, U.K.) for 24 h. At this time, medium was replaced by the collagen/gelatin solutions, with a concentration of 1:15 m/v (collagen solution:medium culture). Latex was used as positive control for cell death, and cell culture medium was used as a negative control representing the ideal situation for cell proliferation. Cell viability was evaluated by assessment of cell metabolic activity using the MTS assay after 72 h in culture. The bioreduction of MTS yields a water-soluble brown formazan product. This was quantified by UV-spectroscopy, reading the formazan absorbance at 490 nm in a microplate reader (Synergy HT, Bio-Tek Instruments, U.S.A.).

The present disclosure relates to a method for obtaining collagen from a marine sponge, namely isolation and/or purification. This method comprises the extraction of the collagen from said sponge using water acidified with carbon dioxide with a pressure between 10 - 60 bar - preferably 10-50 bar - for 3-24 hours, wherein the extraction temperature is between 33 - 37 °C.

The extraction of sponge origin collagen/gelatin with high pressure carbon dioxide-acidified water improved strongly the collagen extraction yields over the values of the prior art - 0.4 - 2%. This new process surprisingly increased the collagen yield for yield values around 10% as observed in table 1, this is an increase of at least 5 times more of collagen extraction yields.

| Table 1 - Extraction yields (%) and collagen content (%) for the different extraction times and pressures used for the marine sponge *Chondrosia reniformis* with the disclosed process. The results are presented as mean ± standard deviation | | | |
|---|---|---|---|
| Conditions (Extraction time/Pressure) | Extraction Yield (%) | Protein Content (%) | Collagen Content (%) |
| 3h / 10 bar (20 °C) | 6.2 | - | - |
| 3h / 10 bar (37 °C) | 11.0 (1.6) | 24.2 (1.8) | 22.8 (5.4) |
| 3h / 50 bar (37 °C) | 9.0 (1.1) | 31.2 (6.6) | 20.5 (0.5) |
| 13.5h / 30 bar (37 °C) | 12.4 (0.3) | 22.3 (3.2) | 20.5 (2.5) |
| 24h / 10 bar (37 °C) | 11.4 (3.0) | 31.1 (9.5) | 30.4 (0.8) |
| 24h / 50 bar (37 °C) | 10.8 (0.8) | 32.5 (3.3) | 28.6 (9.5) |

The statistical analysis of the data was performed using IBM SPSS Statistics Version 20. Normality was verified by the Shapiro-Wilk test. Normal distributed data were then analyzed using one-way ANOVA with a Bonferronís post-test. When normality was not observed, a nonparametric test, namely, Kruskall-Wallis test was performed. Differences between the groups with p < 0.05 were considered to be statistically significant.

In an embodiment, the method further comprises the following steps:
- cutting the marine sponge until a size particle inferior to 1 cm;
- salt leaching the marine sponge, in particular using water, for 1.5 h, and preferably until the solution reaches to conductivity inferior to 12 µS/cm at 20 °C;
- milling the marine sponge to micrometre scale particles, preferably inferior to 500 µm;
- extracting the mixture in a high pressure device;
- separation of the obtained extract, preferably for 0.5 h;
- drying the separated extract, preferably for 12 - 18 h.

The conductivity of the water may be measured by many conventional methods. In this case, the conductivity of the water after washing the marine sponge was determined using a probe (WTW TetraCon 325). The marine sponge samples were salt leached until the final conductivity of the water, for example 80 ml, was at least 12 µS/cm at 20 °C, in particular 11.4 µS/cm at 20 °C or 11.5 µS/cm at 20 °C.

In an embodiment, the extraction pressure of the method herein disclosed is between 30 - 50 bar.

In an embodiment, the extraction of collagen/gelatin is performed for 3 - 15 h, more preferably 10 - 13.5 h.

In an embodiment, the extraction of collagen/gelatin is performed for 13.5 h, at 30 bar and at 37 °C.

In an embodiment, the extraction of collagen/gelatin is
performed for 3 h - 24 h, at 10 bar and at 37 °C.

In an embodiment, ratio of marine sponge/ water during the leaching step is between 2:1 - 1:2, preferably 1:1.

In an embodiment, the separation step of the extract obtained by the method herein disclosed is a filtration step.

In an embodiment, the filtration step comprises two additional steps, a first step to remove solid particles from an extract and a second step to purify the collagen/gelatin extract. In particular, a vacuum filtration system followed by a second filtration step using a 0.45 µm or a 0.22 µm filter.

In an embodiment, the method may also have a step of frozen the obtained collagen.

In an embodiment, the marine sponge is selected from a list consisting of the genus: *Thymosia, Chondrilla* or *Chondrosia.*

In an embodiment, the marine sponge is selected from a list consisting of the species *Thymosia guernei, Chondrilla nucula* and *Chondrosia reniformis.*

In an embodiment, the high pressure device is a high pressure vessel or a high pressure reactor.

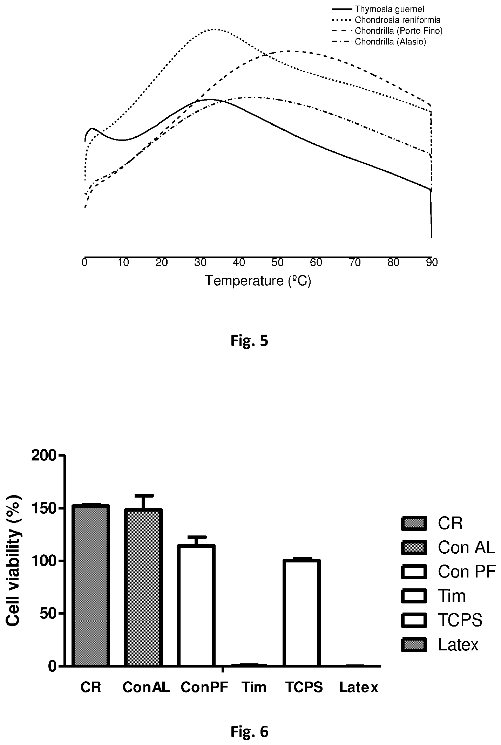

Another aspect of the disclosed subject matter relates to the viability of the cells cultured in a tissue culture plate in the presence of the collagen/gelatin obtainable by the process of the present disclose. The viability of the cells cultured was determined as a function of the cells cultured in the DMEM culture medium. Figure 6 presents the cell viability after 72 h in contact with the material. The obtained results were compared to cell growth on the tissue culture plate in the absence of sponge extract, as positive control, and latex, which was used as negative control. The results show that collagen/gelatin extracted from *Chondrosia reniformis* and *Chondrilla nuculla* (Alassio and Portofino) do not compromise the metabolic activity of the cells. On the contrary, cell viability is higher than 100%, which indicates an increase in the metabolic activity of the cells in the presence of collagen obtainable for the process of the disclosed subject matter . The only exception was the extract from *Thymosia* sp. Even though 82% of the obtained extract was quantified to be collagen/gelatin, there may be some cytotoxic compounds in the extract responsible for the results obtained. Further purification steps in this case would be required to overcome the toxicity observed.

### Brief description of the figures

For a better understanding of the solution, the attached figures are joined, which represent preferred embodiments of the solution. The figures provide preferred embodiments for the present disclosure and should not be seen as limiting the scope of the disclosure.

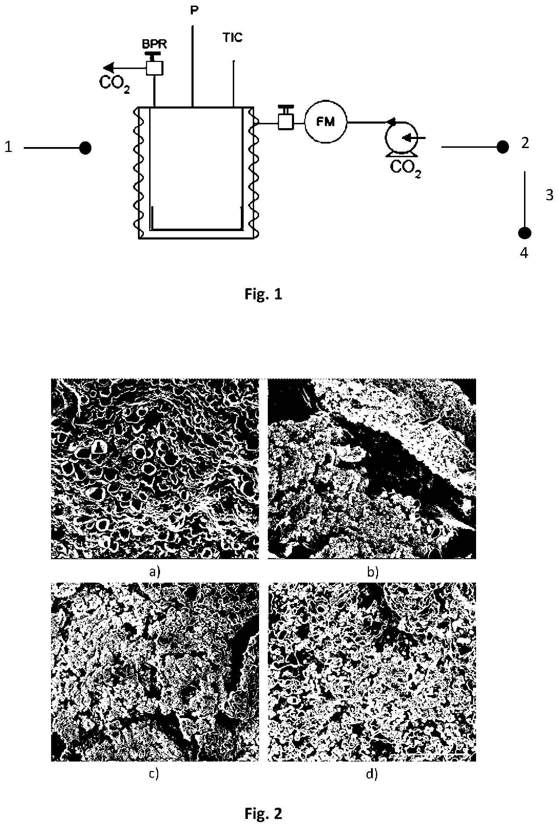

**Figure 1****:** Schematic representation of the extraction procedure for marine sponge collagen/gelatin wherein
   - 1 represents the marine sponge and water in a ratio 1:2 (g/ml);
   - 2 represents the filtration of the extract with a 0.45 µm filter and the freeze of the sample;
   - 3 represents the freeze drying;
   - 4 represents the marine sponge collagen powder;
   - BPR represents the back pressure regulator;
   - P represents the pressure transducer;
   - TIC represents the temperature controller;
   - FM represents the flow meter.
**Figure 2****:** SEM images of the marine sponge collagen/gelatin extracts of (a) TIM, (b) CR, (c) ConAL, and (d) ConPF. Bar = 5 µm.
**Figure 3****:** Fourier transform infrared spectra of the marine sponge collagen/gelatin extracts.
**Figure 4****:** CD spectra of the marine sponge collagen/gelatin extracts measured at room temperature.
**Figure 5****:** DSC thermogram of marine sponge collagen/gelatin extracts.
**Figure 6****:** Cytotoxicity screening of the different extracts from marine sponges. Latex extract and standard culture medium (TCPS) were used as positive and negative controls, respectively.

### Detailed description

The disclosure subject matter relates to a new extraction methodology for the extraction of acid soluble collagen/gelatin, which normally denatures at high temperatures (human collagens) under mild operating conditions, in which water is acidified with carbon dioxide to promote the extraction of collagen/gelatin from different marine sponge species this new process has surprisingly a higher collagen extraction yield.

An embodiment of the present disclosure relates to the use of water acidified with carbon dioxide, for example, at 50 bar to extract collagen/gelatin from the species of marine sponges mentioned. In Figure 1, a schematic representation of the extraction procedure is represented.

An embodiment of the process now disclosed comprises the following steps: washing, milling, extraction, filtration and freeze drying, in particular:
- cutting the marine sponge until a size particle lower than 1 cm;
- salt leaching the marine sponge, in particular using water and for 1.5 h, and preferably until the solution (with a volume of 80 ml) reaches to conductivity inferior to 12 µS/cm at 20 °C, in particular 11.5 µS/cm;
- milling the marine sponge to micrometre scale particles, preferably inferior to 500 µm and preferably for 0.5 h;
- extracting the sponge powder in a high pressure device;
- separation of the obtained extract, preferably for 0.5 h;
- drying the separated extract, preferably for 12 - 18 h.

In another embodiment, the salt leaching of the marine sponge is performed until a conductivity of 12 µS/cm at 20 °C, in particular 11.5 µS/cm, for example in 80 ml of water, is reached.

In another embodiment, the removing step of the solid particles is performed by double filtration using first a vacuum filtration system followed by a second filtration step using a 0.22 µm filter.

The quantification of the extraction yield was calculated as the mass of extract obtained per gram of sponge extracted. The extraction yields are presented in Table 2.

| Table 2 - Extraction Yield and Collagen/Gelatin Content on the Different Extracts | | |
|---|---|---|
| source | gram extract/100 g sample^{a} | collagen/gelatin content (%)^{b} |
| *Thymosea* sp. | 16.6 | 82 |
| *Chondrosia reniformis* | 11.6 | 36 |
| *Chondrilla nuculla* (Alassio) | 9.0 | 54 |
| *Chondrilla nuculla* (Portofino) | 17.3 | 82 |
| ^{a}Values are indicated as a percentage of gram of collagen/gelatin per gram of wet tissue. ^{b}Assessed by Sircol collagen quantification kit. | | |

In an embodiment, the results demonstrate that the lowest extraction yields were obtained for the *Chondrilla nuculla* (Alassio) sponge, and the highest yield was obtained from *Chondrilla nuculla* (Portofino). Regarding the extraction of the acid-soluble fraction from marine sponges from dilute acetic acid solutions, the proposed technology represents an improvement of nearly 30% in extraction yield (Swatschek, D.; Schatton, W.; Kellermann, J.; Kreuter, J. R. Eur. J. Pharm. Biopharm. 2002, 53, 107-113). Besides the determination of the extraction yield, it is also important the quantification of the collagen/gelatin present in the extracts. The quantification of the amount of collagen/gelatin present in each extract was performed using a specific detection kit (Sircol assay kit), which revealed that the extracts recovered from *Thymosea* sp. and *Chondrilla nuculla* (Portofino) present a considerable high value of collagen/gelatin near 82%. Considering that the collagen/gelatin content of these species may vary from 30 to 40 wt % (Swatschek, D.; Schatton, W.; Kellermann, J.; Kreuter, J. R. Eur. J. Pharm. Biopharm. 2002, 53, 107-113), the extraction performed represents hereafter an extraction yield of approximately 50% of the collagen/gelatin present in the sponge. Hence, the proposed technology for the extraction of marine sponges could be a valuable source of collagen/gelatin for industrial exploitation. Such industrial exploitation is also dependent on the sustainability of the raw material, which in the case of marine sponges can be addressed by aquaculture.

The obtained results indicated that the amount of collagen/gelatin extracted from the wild sponge (ConPF) is about three times the amount extracted from the cultured one (ConAL). However, the current study did not account for the time at which marine sponges were collected, and the sustainability can be only correctly assessed with a study over time (covering different seasons) and embracing multiple sponge generations.

In an embodiment, the morphology of the obtained powder was observed by scanning electron microscopy, and the respective images are presented in Figure 2. From the images presented, it is noticeable that the typical fibrillar structure of collagen/gelatin was not observed in any of the samples obtained. However, nodular collagen has been reported in the literature for collagen extracted from *Chondrosia reniformis.*

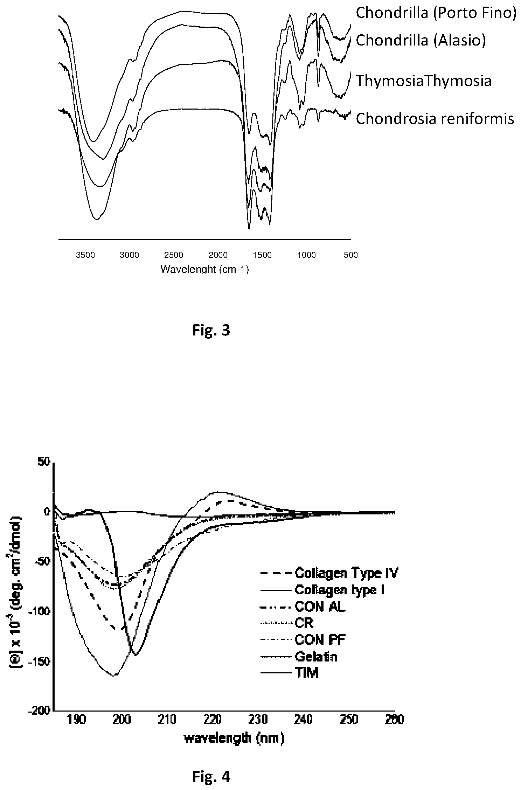

In an embodiment, the representative FTIR spectra of the four marine sponges extracted (TIM, CR, ConAL, and ConPF) in the 4000-400 cm⁻¹ wavenumber regions are presented in Figure 3. The main bands are labelled in the figure and are described in Table 3.

| Table 3 - General Peak Assignments of FTIR Spectra Consist of Marine Sponge Collagen/Gelatin Extracts | | | | |
|---|---|---|---|---|
| ConPF | ConAL | TIM | CR | region peak assignments |
| 3425 | 3425 | - | 3425 | amide A: N-H stretching (in proteins) |
| 3294 | 3294 | 3283 | 3277 | N-H stretching when involved in hydrogen bonding |
| 3082 | 3085 | 3076 | 3080 | amide B: CH₃ asymmetric stretching |
| 2934 | 2931 | 2929 | 2925 | amide B: CH2 asymmetric stretching |
| 2870 | 2873 | 2873 | 2873 | CH₃ asymmetric bending |
| 2835 | - | - | 2853 | CH₃ symmetric stretching |
| 1653 | 1647 | 1653 | 1644 | amide I: C=O stretching (in proteins) |
| 1522 | 1522 | 1522 | 1522 | amide II: N-H bending |
| 1242 | 1240 | 1240 | 1249 | amide III: C-H stretching |
| 1078 | 1075 | 4080 | 1080 | PO₂ symmetric stretching |
| 1034 | 1034 | 1046 | 1040 | C-O-H from carbohydrates |

In an embodiment, the FTIR spectra of marine sponge extracts are relatively complex and comprise several bands corresponding not only to collagen chemical groups but also probably to other proteins, lipids carbohydrates, and nucleic acids, as shown in Figure 3.

In an embodiment, the Fourier transform infrared spectra of collagen/gelatin extracted from *Thymosia* sp., *Chondrosia reniformis,* and *Chondrilla nuculla* (Alasio and Portofino) marine sponges had great similarity to each other, which suggested their chemical compositions were relatively similar. The FTIR spectra of different collagen/gelatin contained several bands representing amide A, amide B, amide I, amide II, and amide III, which were similar to those collagen/gelatin from other marine sources. The amide A band is generally associated with the N-H stretching vibration and shows the existence of hydrogen bonds and in the extracts it was found at 3425 cm⁻¹. The amide B band was observed at 3283, 3277, 3294, and 3294 cm⁻¹ for TIM, CR, ConAL, and ConPF, respectively, which represents the asymmetrical stretch of CH₃. In an embodiment, the amide I band, associated with stretching vibrations of the carbonyl groups (C=O bond) (Pallela, R.; Bojja, S.; Janapala, V. R. Int. J. Biol. Macromol. 2011, 49, 85-92), was observed at 1653, 1644, 1647, and 1653 cm⁻¹ for TIM, CR, ConAL, and ConPF, respectively. The amide II band appeared at 1522 cm⁻¹ for all extracts and is a result of the N-H bending vibration coupled with C-N stretching vibration. Amide III was observed at 1240, 1249, 1240, and 1242 cm⁻¹ for TIM, CR, ConAL, and ConPF, respectively. Furthermore, the presence of the amide III (C-H stretching) observed by IR absorption suggests the helical structure of the collagen/gelatin extracted. The marine collagen/gelatin extracted with CO₂ acidic water showed a secondary structure.

In an embodiment, the circular dichroism spectra (CD) of the four marine sponges extracted (TIM, CR, ConAL, and ConPF) in the wavelength range of 180-260 nm are shown in Figure 4. CD spectra of the collagen/gelatin controls (collagen type I from bovine and collagen type IV from human placenta) present two peaks, a positive peak at 221 nm and a negative peak at 192 nm. This is a characteristic profile of the collagen triple helix. On the other hand, gelatin, used as a control, and the extracted material from ConAL, ConPF, and CR do not induce positive peaks (220 nm), suggesting the existence of random coils. The negative peak of the extracted materials is present close to 192 nm, around the value of the negative peak for collagen type IV. In the case of TIM, the CD spectra is not conclusive due the presence of other peaks that may suggest the presence of others compounds.

In an embodiment, the denaturation temperature of collagen/gelatin was determined by differential scanning calorimetry. The marine sponge collagen/gelatin extracted had different denaturation temperatures (Td) depending on the source (Figure 5 and Table 4). The Td of TIM (31.02 °C) and CR (30.48 °C) are similar, but the Td values of ConPF (50.05 °C) and ConAL (38.93 °C) show higher values.

| Table 4 - Denaturation temperature of the different sponge extracts | |
|---|---|
| Source | dehydrated samples, T (°C) |
| *Thymosea guernei*^{a} | 31.02 |
| *Chondrosia reniformis^{a}* | 30.48 |
| *Chondrilla nuculla* (Alasio)^{a} | 38.93 |
| *Chondrilla nuculla* (Portofino)^{a} | 50.05 |
| ^{a} Onset point determination. | |

Interestingly, comparing the Td of *Chondrilla nuculla,* the species cultivated in aquaculture (Alassio) to the one harvested in the wild (Portofino) present different values. The value observed for ConPF is the higher reported in the literature. The TIM and CR denaturation temperatures are near collagen type IV from human placenta (28.5 °C). The Td of the collagen/gelatin extracted from the marine sponges, depending on the source, was close to the other marine sources like muscle of carp (32.50 °C) and Japanese sea bass (30.00 °C) and also calf skin collagen (40.00 °C) as reported in the literature. The results suggest that intramolecular hydrogen bonds stabilizing the triple helix structure of collagen might be disrupted to some levels, mainly due to the repulsion of collagen molecule in acidic solution. This phenomenon was observed by different authors in extraction of collagen in various marine sources.

In an embodiment, the content of the amino acids proline and hydroxyproline has been correlated with the thermal denaturation temperature of marine origin collagen. The amino acid composition for the three marine sponges collagen/gelatin extracted was thus determined. The amino acid composition of collagen/gelatin from TIM, CR, ConAl, and ConPF had similar amino acid profiles between them and are presented in Table 5.

| Table 5. Amino Acid Composition of Different Sponge Extracts (residues/1000 residues) | | | | |
|---|---|---|---|---|
| amino acid | TIM | CR | ConAL | ConPF |
| Ala | 101 | 118 | 118 | 113 |
| Thr | 78 | 58 | 61 | 69 |
| Ser | 61 | 59 | 33 | 30 |
| Glu | 117 | 60 | 89 | 85 |
| Gly | 149 | 235 | 239 | 237 |
| Asp | 69 | 66 | 48 | 43 |
| Cys | 5 | 3 | 3 | 4 |
| Val | 29 | 40 | 25 | 26 |
| Met | 4 | 6 | 3 | 2 |
| Ile | 11 | 16 | 9 | 10 |
| Leu | 26 | 22 | 20 | 22 |
| Tyr | 3 | 4 | 7 | 7 |
| Phe | 17 | 15 | 11 | 9 |
| Lys | 30 | 19 | 18 | 16 |
| His | 2 | 6 | 4 | 5 |
| Lys | 22 | 27 | 24 | 21 |
| Arg | 36 | 33 | 39 | 33 |
| Ohpro | 81 | 60 | 83 | 93 |
| Pro | 160 | 151 | 166 | 175 |
| imino acid | 241 | 211 | 249 | 268 |

Comparing these results with other collagen/gelatin compositions from marine sources, we observed an analogous behavior. Collagen is composed by a triple helical sequence of amino acid repeat, (Gly-Pro-Hyp)ₙ, the most abundant of which is glycine (Gly). This was observed in all extracts where the amounts of 149, 235, 239, and 237 glycine residues per 1000 total amino acid residues were determined. Considering the imino acid content (Proline (Pro) + hydroxiproline (Hyp)), the results show values around ∼240 total amino acid residues per 1000 residues, which was slightly higher than in the case of others marine sources (Swatschek, D.; Schatton, W.; Kellermann, J.; Kreuter, J. R. Eur. J. Pharm. Biopharm. 2002, 53, 107-113) and can also justify the higher values of denaturation temperatures. The imino acid contributes to the formation and stabilization of cross-links in the collagen. The alterations in imino acid content are associated with the different living environments, particularly the temperature of the habitat.

The isoelectric point is an important parameter of proteins because it is related with amino acid content. In an embodiment, all the samples present a similar behavior, showing an isoelectric point around pH 8, in the basic range due to the acidic conditions of the extraction, because this maintained intact the amide residues. This observation has been reported by Swatschek for collagen extracted from *Chondrosia reniformis,* who indicates an isoelectric range between 6.5 and 8.5 (Swatschek, D.; Schatton, W.; Kellermann, J.; Kreuter, J. R. Eur. J. Pharm. Biopharm. 2002, 53, 107-113). The titration curves show a flattening area at pH 6 that eventually levels off at pH 2. The behavior of the extracts present a second critical point noted at pH 5, common to the four samples of collagen/gelatin. The existence of two different critical points in the pH mobility of collagen and gelatin has been reported before by Highberger et al. (Highberger, J. H. J. Am. Chem. Soc. 1939, 61, 2302-2303.), namely at pH of 4.7 and 7.7.

In an embodiment, it is suggested the presence, in the extracts, of a mixture between collagen and gelatin.

The molecular weight of the extracts was determined, in particular, by size exclusion chromatography (GPC-SEC). The molecular weight of a substance, particularly a polymer, is a key chemical characteristic that can dramatically influence the material mechanical performance, particularly the viscosity and rheological behavior. In this sense, size exclusion chromatography was used to determine the number-average molecular weight (Mₙ) and weight-average molecular weight (_{Mw}) for the four different collagens extracted (Table 6). Higher Mn and Mw were obtained in collagen/gelatin extracted from *Chondrosia reniformis* with 60.57 and 208.92 kDa, respectively. The molecular weight of the extracts is similar to the one of collagen/gelatin extracted from other marine sources, such as eel fish skin (∼95 kDa/∼210 kDa), skin of strip catfish (∼26 kDa/∼95 kDa), and calf skin collagen and skin of largefin longbarbel catfish (∼116 kDa/∼200 kDa).

| Table 6. Mₙ and M_{w} of Different Sponge Extracts ^{a} | | |
|---|---|---|
| sample | Mₙ (kDa) | Mw (kDa) |
| *Thymosea* sp. | 48.98 (±10.63) | 155.40 (±20.42) |
| *Chondrosia reniformis* | 60.57 (±15.73) | 208.92 (±14.96) |
| *Chondrilla nuculla* (Portofino) | 34.29 (±6.52) | 112.77 (±12.60) |
| *Chondrilla nuculla* (Alassio) | 34.29 | 110.59 (±8.75) |
| ^{a} Standard deviations are in parentheses. | | |

The values of the molecular weight reported in the literature are very wide. This is mostly due to the fact that the extraction processes have a high impact on the final extracts. The result of the molecular weight of collagen/gelatin extracted from marine sponges by CO₂ acidic water at room temperature demonstrated that they could be used as appropriate materials for biomaterial applications.

The eventual cytotoxicity of the four different collagen/gelatin extracts obtained were evaluated in accordance with the protocol described in ISO/EN 10-993.27

The extraction of sponge origin collagen/gelatin with high pressure carbon dioxide-acidified water was successfully achieved. The proposed methodology allows an extraction of nearly 50% of the collagen/gelatin content of the sponges tested, representing an increase of more than 30% over conventional acid extractions using dilute acetic acid solutions. The extracted material was confirmed to be a mixture of collagen and gelatin by different physical and chemical analysis techniques. Cytotoxicity behaviour demonstrated that the collagen/gelatin obtained is non-cytotoxic. The results presented suggest that sponge collagen extracted with water acidified by carbon dioxide is a promising material for biomedical applications.

The present invention is not, obviously, in any way restricted to the herein described embodiments and a person with average knowledge in the area can predict many possibilities of modification of the same invention and substitutions of technical characteristics by others equivalent, depending on the requirements of each situation, as defined in the appended claims.

The embodiments described above can be combined with each other. The following claims further define the preferred embodiments of the present invention.

## Claims

1. A method for obtaining collagen/gelatin from a marine sponge comprising the extraction of the collagen/gelatin from said sponge using water acidified with carbon dioxide with a pressure between 10 - 60 bar, during 3-24 hours, wherein the extraction temperature is between 33 - 37 °C.

2. Method according to the previous claim further comprising the following steps:
cutting the marine sponge until a size particle inferior to 1 cm;
salt leaching the marine sponge, in particular using water, for 1.5 h, and preferably until the solution reaches to conductivity inferior to 12 µS/cm at 20 °C;
milling the marine sponge to micrometre scale particles, preferably inferior to 500 µm;
extracting the mixture in a high pressure device
separation of the obtained extract, preferably for 0.5 h;
drying the separated extract, preferably for 12 - 18 h.

3. Method according to the previous claims wherein the extraction pressure is between 30 - 50 bar.

4. Method according to the previous claims wherein the extraction step is between 3 - 15 h, more preferably 10 - 13.5 h.

5. Method according to the previous claims wherein the extraction is performed for 13.5 h, at 30 bar and at 37 °C.

6. Method according to the previous claims wherein the extraction is performed for 3 h - 24 h, at 10 bar and at 37 °C.

7. Method according to the previous claims wherein the ratio of marine sponge/ water during the leaching step is between 2:1 - 1:2, preferably 1:1.

8. Method according to the previous claims wherein the separation step of the obtained extract is a filtration step.

9. Method according to the previous claim wherein the filtration step that comprises two additional steps, a first step to remove solid particles from an extract and a second step; in particular a vacuum filtration system followed by a second filtration step using a 0.45 µm filter.

10. Method according to the previous claims wherein the marine sponge is selected from a list consisting of the genus: *Thymosia, Chondrilla* or *Chondrosia.*

11. Method according to the previous claims wherein the marine sponge is selected from a list consisting of the species *Thymosia guernei, Chondrilla nucula* and *Chondrosia reniformis.*

## Patentansprüche

1. Ein Verfahren zur Gewinnung von Kollagen/Gelatine aus einem Meeresschwamm, umfassend die Extraktion des Kollagens/der Gelatine aus dem genannten Schwamm unter Verwendung von mit Kohlendioxid angesäuertem Wasser mit einem Druck zwischen 10 - 60 bar während 3-24 Stunden, wobei die Extraktionstemperatur zwischen 33 - 37 °C liegt.

2. Verfahren nach dem vorangehenden Anspruch, ferner die folgenden Schritte umfassend:
Zerschneiden des Meeresschwamms auf eine Partikelgröße von weniger als 1 cm;
Auswaschen des Salzes aus dem Meeresschwamm, insbesondere mit Wasser, für 1,5 Std., und vorzugsweise bis die Lösung eine Leitfähigkeit von weniger als 12 µS/cm bei 20 °C erreicht;
Zermahlen des Meeresschwammes zu Mikrometer großen Partikeln, vorzugsweise weniger als 500 µm;
Extrahieren des Gemisches in einer Hochdruckvorrichtung
Trennen des erhaltenen Extrakts, vorzugsweise für 0,5 Std.;
Trocknen des abgetrennten Extrakts, vorzugsweise für 12 - 18 Std.

3. Verfahren nach den vorangehenden Ansprüchen, wobei der Extraktionsdruck zwischen 30 - 50 bar liegt.

4. Verfahren nach den vorhergehenden Ansprüchen, wobei der Extraktionsschritt zwischen 3 - 15 Std., besonders bevorzugt 10 - 13,5 Std. liegt.

5. Verfahren nach den vorangehenden Ansprüchen, wobei die Extraktion für 13,5 Std. bei 30 bar und 37 °C erfolgt.

6. Verfahren nach den vorangehenden Ansprüchen, wobei die Extraktion für 3 Std. - 24 Std. bei 10 bar und 37 °C erfolgt.

7. Verfahren nach den vorangehenden Ansprüchen, wobei das Verhältnis von Meeresschwamm / Wasser während des Auswaschschritts zwischen 2:1 - 1:2, bevorzugt 1:1, liegt.

8. Verfahren nach den vorangehenden Ansprüchen, wobei der Trennschritt für das erhaltene Extrakt ein Filterschritt ist.

9. Verfahren nach dem vorangehenden Anspruch, wobei der Filterschritt zwei zusätzliche Schritte umfasst, einen ersten Schritt zum Entfernen von Festpartikeln aus einem Extrakt und einen zweiten Schritt; insbesondere ein Vakuumfiltrationssystem, gefolgt von einem zweiten Filterschritt unter Verwendung eines 0,45 µm Filters.

10. Verfahren nach den vorangehenden Ansprüchen, wobei der Meeresschwamm aus einer Liste der Gattungen ausgewählt wird: *Thymosia, Chondrilla* oder *Chondrosia.*

11. Verfahren nach den vorangehenden Ansprüchen, wobei der Meeresschwamm aus einer Liste der Arten *Thymosia guernei, Chondrilla nucula* and *Chondrosia reniformis* ausgewählt wird.

## Revendications

1. Une méthode pour obtenir du collagène/ de la gélatine à partir d'une éponge marine comprenant l'extraction du collagène/ de la gélatine de ladite éponge utilisant de l'eau acidifiée avec du dioxyde de carbone avec une pression située entre 10-60 bar, durant 3-24 heures, dans laquelle la température d'extraction est située entre 33-37ºC.

2. Méthode selon la revendication précédente comprenant également les étapes suivantes :
couper l'éponge marine jusqu'à obtenir une particule de taille inférieure à 1 cm ;
lixivier le sel de l'éponge marine, en particulier en utilisant de l'eau, durant 1.5 h, et de préférence jusqu'à ce que la solution atteigne une conductivité inférieure à 12 µS/cm à 20ºC ;
moudre l'éponge marine en particules à l'échelle du micromètre, de préférence inférieures à 500 µm ;
extraire le mélange dans un dispositif à haute pression
séparation de l'extrait obtenu, de préférence durant 0.5 h ;
sécher l'extrait séparé, de préférence durant 12-18 h.

3. Méthode selon les revendications précédentes dans laquelle la pression d'extraction est située entre 30-50 bar.

4. Méthode selon les revendications précédentes dans laquelle l'étape d'extraction est située entre 3-15 h, de préférence entre 10-13.5 h.

5. Méthode selon les revendications précédentes dans laquelle l'extraction est effectuée durant 13.5h, à 30 bar et à 37ºC.

6. Méthode selon les revendications précédentes dans laquelle l'extraction est effectuée durant 3h-24h, à 10 bar et à 37ºC.

7. Méthode selon les revendications précédentes dans laquelle le ratio éponge marine/eau durant l'étape de lixiviation est situé entre 2 :1-1 :2, de préférence 1 :1.

8. Méthode selon les revendications précédentes dans laquelle l'étape de séparation de l'extrait obtenu est une étape de filtration.

9. Méthode selon la revendication précédente dans laquelle l'étape de filtration comprend deux étapes supplémentaires, une première étape pour retirer les particules solides d'un extrait et une seconde étape ; en particulier un système de filtration sous vide suivi d'une seconde étape de filtration utilisant un filtre de 0.45 µm.

10. Méthode selon les revendications précédentes dans laquelle l'éponge marine est sélectionnée à partir d'une liste constituée des genres : *Thymosia, Chondrilla* ou *Chondrosia.*

11. Méthode selon les revendications précédentes dans laquelle l'éponge marine est sélectionnée à partir d'une liste constituée des espèces *Thymosia guernei, Chondrilla nucula* et *Chondrosia reniformis.*
